(19) Europäisches Patentamt
European Patent Office
Office européen des brevets

(11) **EP 2 502 567 A1**

(12) **EUROPEAN PATENT APPLICATION**
published in accordance with Art. 153(4) EPC

(43) Date of publication:
26.09.2012 Bulletin 2012/39

(51) Int Cl.:
*A61B 10/00* (2006.01)   *A61B 5/1455* (2006.01)

(21) Application number: **10829975.1**

(22) Date of filing: **11.11.2010**

(86) International application number:
**PCT/JP2010/070079**

(87) International publication number:
**WO 2011/059016 (19.05.2011 Gazette 2011/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.11.2009 JP 2009260550**

(71) Applicant: **Hitachi Medical Corporation Chiyoda-ku Tokyo 101-0021 (JP)**

(72) Inventor: **SUZUKI, Hiromichi Tokyo 101-0021 (JP)**

(74) Representative: **Strehl Schübel-Hopf & Partner Maximilianstrasse 54 80538 München (DE)**

(54) **ORGANISM LIGHT MEASURING DEVICE AND METHOD FOR DISPLAYING INFORMATION RELATING TO NECESSITY/UNNECESSITY OF REPLACEMENT OF LIGHT-EMITTING PART**

(57)  Disclosed is a biological optical measurement instrument provided with: a light source unit which has a light-emitting unit that emits light and irradiates the light to an object; a light measurement unit configured to measure passing light at a measurement point of the object: a signal processing unit configured to process hemoglobin information outputted from the light measurement unit and create a light measurement image; and a display unit configured to display the light measurement image, further provided with a measurement/determination unit configured to measure the deterioration state of the light-emitting unit from the light output of the light-emitting unit and an electric current injected into the light-emitting unit and determine the necessity/unnecessity of replacement of the light-emitting unit, wherein the display unit displays information relating to the necessity/unnecessity of replacing the light-emitting unit determined by the measurement/determination unit.

FIG.10

**Description**

Field of the Invention

The present invention relates to a biological optical measurement instrument which irradiates near-infrared light to an object to be examined and measures the light that has been transmitted through or reflected from inside of the object so as to measure information on hemoglobin in the object, and the method for displaying information relating to necessity/unnecessity of replacement of a light-emitting part.

Description of Related Art

The biological optical measurement instrument is capable of measuring information on hemoglobin inside of the body of an object safely without restricting the object by a fixed belt, etc. by applying probes mounted with a plurality of optical fibers to the object. The probes are formed by irradiating probes that irradiate near-infrared light emitted from light-emitting units such as laser and receiving probes that receive the light which has been transmitted through the object. Also, the measurement result of hemoglobin information is displayed by graphs or grayscale images.
While biological optical measurement instruments measure intensity of the light which is transmitted through an object, since the intensity of light-irradiation from irradiating-probes, etc. can not be inspected, change in irradiation intensity due to deterioration of optical fiber cables has not been detectable.
Given this factor, Patent Document 1 discloses the technique that provides an inspection-body inserting holes that receive irradiating probe application parts and detecting probe application parts, so that the irradiation intensity of the light from irradiation probes can be measured by inserting a light-detecting inspection body or a light-intensity inspection body into the inspection-body inserting hole.

Prior Art Documents

Patent Documents

Patent Document 1: JP-A-2007-209680

However, though light-emitting performance of a light-emitting unit deteriorates with time and eventually disables the measurement performance of biological optical measurement, Patent Document 1 does not consider display of information regarding necessity/ unnecessity of replacing a light-emitting unit for enabling an operator to determine necessity/unnecessity of the replacement.
The objective of the present invention is to provide a biological optical measurement instrument and the method for displaying information regarding necessity/unnecessity of replacing a light-emitting unit capable of displaying information regarding necessity/unnecessity of replacing a light-emitting unit for enabling an operator to determine the necessity/unnecessity of the replacement.

Brief Summary of the Invention

In order to achieve the above-described objectives, the present invention measures deterioration state of a light-emitting unit from the light output of the light-emitting unit which emits light and the current injected into the light-emitting unit, and displays information regarding the necessity/unnecessity of replacement of the light-emitting unit referring to the deterioration state.
Concrete configuration of the present invention will be described in the following paragraph.
(1) The biological optical measurement instrument of the present invention comprising:

a light source unit which has light-emitting units, configured to irradiate the light to an object to be examined;
a light measurement unit configured to measure the transmitted light in the measurement point of the object;
a signal processing unit configured to process the hemoglobin information outputted from the light measurement unit and creates a light measurement image;
a display unit configured to display the light measurement image; and
a measurement/determination unit configured to measure deterioration state of the light-emitting unit from the light output of the light-emitting unit and the current which is injected into the light-emitting unit, and determines the necessity/unnecessity of replacing the light-emitting unit,

wherein the display unit displays the information regarding the necessity/unnecessity of replacement of the light-emitting unit which is determined by the measurement/determination unit.

(2) The method for displaying the information regarding necessity/unnecessity of replacement of the light-emitting unit includes:

a step of measuring the deterioration state of the light-emitting unit from the light output of a light-emitting unit and the current injected into the light-emitting unit; and

a step of displaying the information regarding necessity/unnecessity of replacement of the light-emitting unit based on the deterioration state.

Effect of the Invention

In accordance with the biological optical measurement instrument and the method for displaying information regarding necessity/unnecessity of replacing a light-emitting unit related to the present invention, it is possible to display information regarding the necessity/unnecessity of replacing the light-emitting unit so as to facilitate the determination on necessity/unnecessity of replacement of the light-emitting unit by referring to the deterioration state of the light-emitting performance thereof.

Brief Description of the Diagrams

Fig. 1 shows the general configuration of the present invention.

Fig. 2 shows a measurement device which measures the light output of a light-emitting unit 16 related to the present invention.

Fig. 3 shows a measurement device which measures the light output of the light-emitting unit 16 related to the present invention.

Fig. 4 is a view showing the process of determining deterioration state of the light-emitting unit 16 related to the present invention.

Fig. 5 is a view showing the process of determining deterioration state of the light-emitting unit 16 related to the present invention.

Fig. 6 shows the display pattern of deterioration state of the light-emitting unit 16 related to the present invention.

Fig. 7 shows a second embodiment of the present invention.

Fig. 8 shows a third embodiment of the present invention.

Fig. 9 is a flowchart showing the operation in the first ~ third embodiments of the present invention.

Fig. 10 shows a fourth embodiment of the present invention.

Fig. 11 is a flowchart showing the operation in the fourth embodiment of the present invention.

Fig. 12 is a view showing the pattern that a timer 130 or an ammeter 132 is mounted in the light-emitting unit 16 of the present invention.

Detailed Description of the Invention

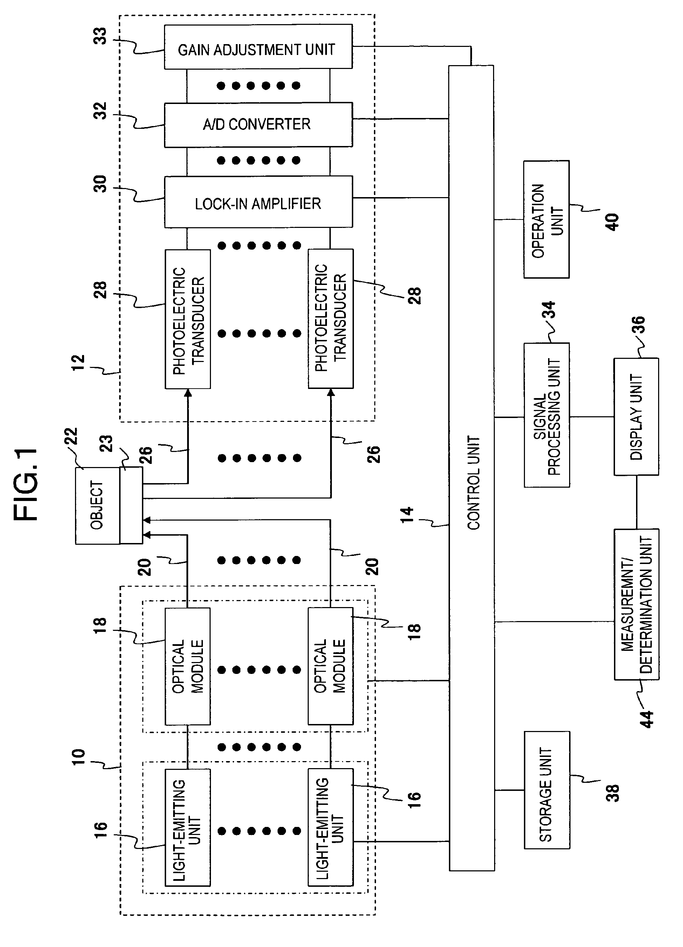

A biological optical measurement instrument irradiates near-infrared light to the inside of the body of an object 22, and detects the light which is reflected from the vicinity of the surface of the object 22 or transmitted through the body of the object 22 (hereinafter referred to as mere transmitted light). This biological optical measurement instrument mainly comprises a light source unit 10 configured to irradiate near-infrared light, a light measurement unit 12 configured to measure the transmitted light and convert the light into an electric signal, a control unit 14 configured to control driving of the light source unit 10 and the light measurement unit 12, and display unit 36 as shown in Fig. 1.

The light source unit 10 comprises a plurality of light-emitting units 16 that emit light having a predetermined wavelength and a plurality of optical modules 18 provided with a modulator that modulates the light which is emitted by the light-emitting unit 16 by a plurality of different frequencies, and the outputted light from the respective optical modules 18 are irradiated to predetermined positions of the object 22 via irradiating optical fibers 20 respectively.

A probe holder 23 is to be applied on the object 22, and a plurality of irradiating optical fibers 20 and detecting optical fiber 26 are detachably attached in the respective setting positions (holes) of the probe holder 23.

The light source unit 10 comprises the number of optical modules 18 corresponding to the number of the light-emitting units 16. While the wavelength of light depends on the spectral characteristic of the target subject in the object 22, in the case that the oxygen saturation or amount of blood is measured from the hemoglobin information of oxygenated hemoglobin and deoxygenated hemoglobin, one or plural wavelengths are selected and used from the light within the wavelength range of 600nm ~ 1400nm. The light-emitting unit 16 in the light source unit 10 is configured to generate the light having two kinds of wavelengths, for example that are 780nm and 830nm, and these two wavelengths of light are synthesized and irradiated from one irradiating position.

The light measurement unit 12 is formed by photoelectric transducers 28 which are photodiode, etc. configured to convert the transmitted light induced from a plurality of measurement positions in the object 22 via detecting optical fibers 26 into the electric quantity corresponding to the light quantity respectively, a lock-in amplifier 30 configured to input the electric signals from the photoelectric transducers 28 and selectively detect the modulated signals corresponding to the light irradiating positions, and an A/D converter 32 configured to convert the output signals from the lock-in amplifier 30 into digital signals based on the hemoglobin information, and a gain adjustment unit 33 configured to multiply the digital signal by the gain value based on the light quantity of the detected transmitted light. The lock-in amplifier 30 selectively detects the modulated signals corresponding to the light irradiating positions and the two wavelengths. The gain adjustment unit 33 multiplies the digital signal outputted from the A/D converter 32 by the gain value on the basis of the electric signal based on the light quantity of the transmitted light which is detected by the plurality of photoelectric transducers 28.

Also, the biological optical measurement instrument comprises a signal processing unit 34 configured to process the digital signal based on the hemoglobin information so as to create a graph of the hemoglobin information for each channel or a light measurement image of the hemoglobin information such as a 2-dimensional or 3-dimensional grayscale image in which the respective digital signals are interpolated for each channel, a display unit 36 configured to display the light measurement image outputted from the signal processing unit 34, a head-surface image, a brain-surface image, measurement points, and so on, a storage unit 38 configured to store the necessary data for the process or the processing result of the signal processing unit 34, a measurement/determination unit 44 configured to measure the deterioration state of the light-emitting unit 16 and determine the necessity/unnecessity of replacement of the light-emitting unit 16 on the basis of the deterioration state, and an operation unit 40 configured to input various commands necessary for operating the instrument.

Embodiment 1

(Current Comparison)

Here, the first embodiment will be described referring to Fig. 1 ~ Fig. 6. In the first embodiment, the measurement/determination unit 44 determines the necessity/unnecessity of the light-emitting unit 16 from the deterioration state of the light-emitting unit 16 acquired from the light output (mW) of the light-emitting unit 16 which is the light-emitting element that emits light and the current (mA) injected into the light-emitting unit 16, and the display unit 36 displays the deterioration state of the light-emitting unit 16 and the information regarding necessity/unnecessity of the replacement thereof. The light-emitting unit 16 emits near-infrared light including semiconductor laser that emits light using induced emission or light-emitting diode that emits light using a semiconductor.

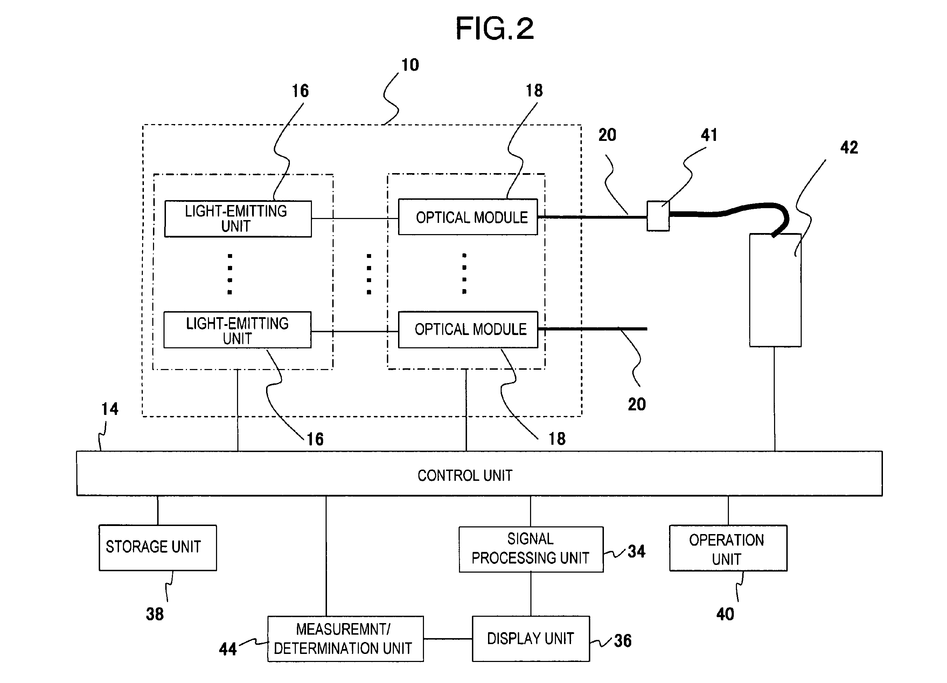

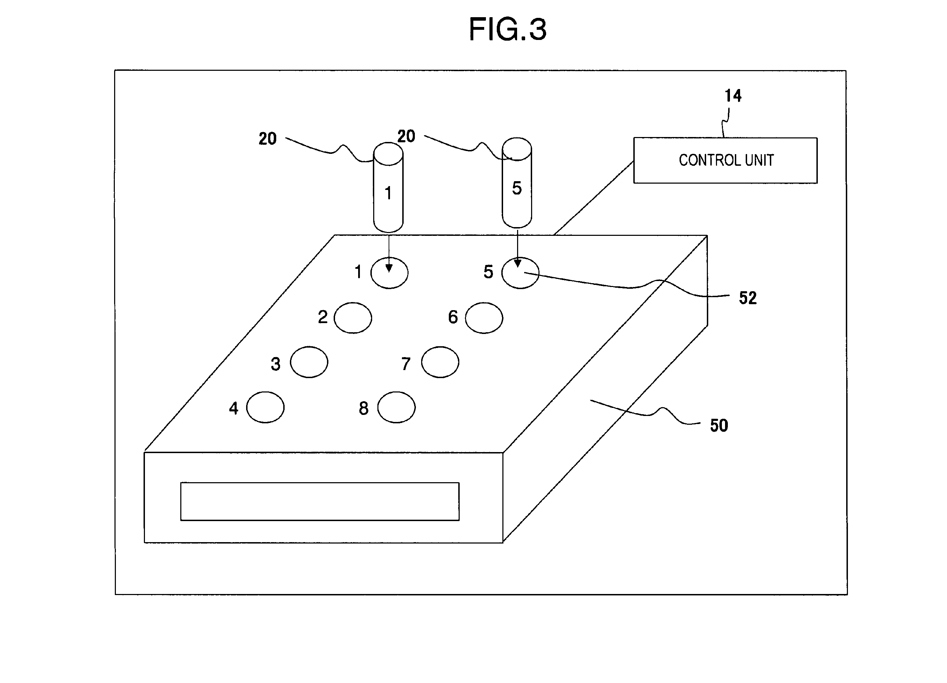

Fig. 2 and Fig. 3 show the measurement device that measures the light output of the light-emitting unit 16. Fig. 2 shows a measurement device that measures the light output of one irradiating optical fiber 20. The light emitted from the light-emitting unit 16 is outputted to the irradiating optical fiber 20 via the optical module 18. To the irradiating optical fiber 20, a light sensor 41 that receives the light outputted from the irradiating optical fiber 20 and passes the current in accordance with the amount of light received is connected. The light sensor 41 is attached at the end of the irradiating optical fiber 20. The light sensor 41 is connected to a light power meter 42. The light power meter 42 converts the current outputted from the light sensor 41 and calculates the light output. The light power meter 42 is connected to the control unit 14, and the calculated light output is transmitted to the measurement/determination unit 44 and the storage unit 38 via the control unit 14.

Fig. 3 shows the measurement device that measures the light output of a plurality of irradiating optical fibers 20. The main body of the biological optical measurement instrument is provided with a holder-inserting hole (not shown in the diagram). Into the holder-inserting hole, a tabular inspection holder 50 is inserted. The inspection holder 50 is pulled out of the main body of the biological optical measurement instrument at the time of inspection, and is pushed into and stored in the main body of the instrument when the inspection is not performed. More specifically, the inspection holder 50 is horizontally slidable between the inspection position and the stored position.

Fiber-inserting holes 52 of the inspection holder 50 comprises a plurality of light sensors 41 that receive the light outputted from the irradiating optical fibers 20 and pass the current in accordance with the amount of light received and light power meters 42 that convert the current outputted from the light sensor 41 and calculate the light output. In Fig. 3, the light sensors 41 and the light power meters 42 are respectively provided to eight fiber-inserting holes 52. The light power meters 42 are connected to the control unit 14, and the calculated light output is transmitted to the measurement/determination unit 44 and the storage unit 38 via the control unit 14.

On the top surface of the inspection holder 50, the number corresponding to the number of the irradiating optical fibers 20 is listed near the fiber-inserting holes 52 into which the irradiating optical fibers 20 are inserted. For example, the irradiating optical fiber 20 of number "5" is inserted into the fiber-inserting hole 52 of number "5", and the light output of the light-emitting unit 16 corresponding to the irradiating optical fiber 20 of number "5" is calculated by the light sensor 41 and the light power meter 42. The calculated light output is transmitted to the measurement/ determination unit 44 and the storage unit 38 via the control unit 14 along with the number thereof.

The control unit 14 transmits the light output to be outputted from the light power meter 42 and the current to be injected into the light-emitting unit 16 which is necessary for the light output to the measurement/determination unit 44 and the storage unit 38. The measurement/determination unit 44 measures the deterioration state of the light-emitting unit 16 by the light output and the current. Then the measurement/determination unit 44 determines the necessity/ unnecessity of replacing the light-emitting unit 16 on the basis of the deterioration state of the light-emitting unit 16. The display unit 36 displays the deterioration state of the light-emitting unit 16 outputted from the measurement/determination unit 44 and the information regarding the necessity/unnecessity of replacement. The storage unit 38 stores the relevant light output and current along with the time information (for example, the date and time).

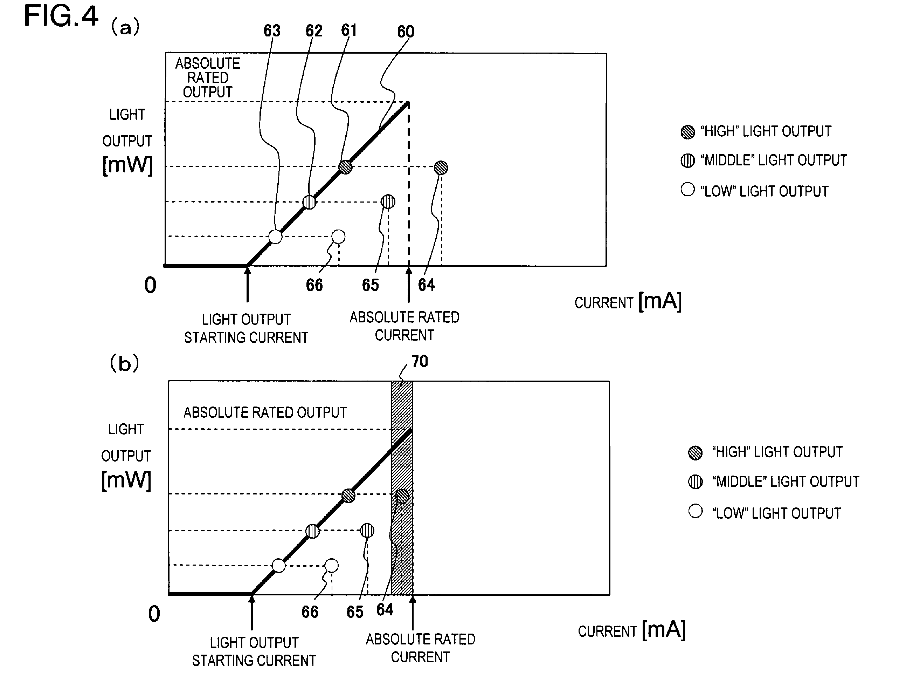

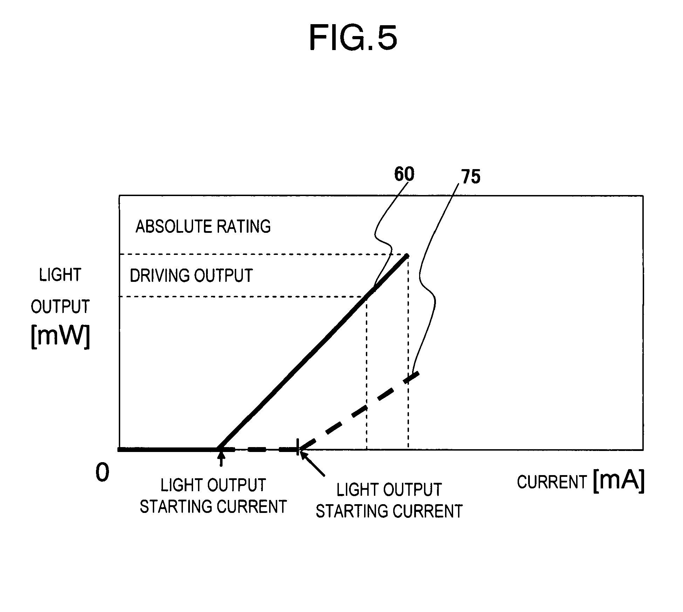

The procedure for measurement of deterioration state of the light-emitting unit 16 and determination of the necessity/unnecessity of replacement will be described using Fig. 4 ~ Fig. 6. Figs. 4(a), (b) and Fig. 5 show the relationship between the light output which is outputted from the light power meter 42 and the current necessary for the light output.

The biological optical measurement instrument used in the present invention is capable of executing three different intensities of light output that are "high", "middle" and "low". The "high" light output is a normal light output to be used in the normal measurement mode, and is used for general adults. The "middle" light output and "low" light output are particular kinds of light output to be used for the measurement modes wherein the light output is reduced for performing an examination mainly on infants and persons through which light transmits easily. In the present invention, for example, "High" is set as 2(mW), "Middle" is set as 1(mW) and "Low" is set as 0.5(mW).

A graph 60 in Fig. 4 shows the relationship between the light output of the light-emitting unit 16 at the time of shipment of a biological optical measurement instrument, i.e. at a normal and initial condition of the instrument. The light output and the current of the biological optical measurement instrument at the time of shipment is stored in the storage unit 38 in advance. The measurement/determination unit 44 creates the graph 60 by reading out the data of the light output and the current of the biological optical measurement instrument at the time of shipment, and causes the display unit 36 to display the graph 60. A "high" light output 61, a "middle" light output 62 and a "low" light output 63 of the biological optical measurement instrument at the time of shipment are marked on the graph 60.

In the graph 60, when the applied current is smaller than a predetermined value, the light output here becomes 0 since the light is not emitted from the light-emitting unit 16 in the range between the current of 0 and a predetermined light-output starting current. When the current which is more than the light-output starting current is applied, the light output can be acquired. The more the current is increased, the light output is also increased.

A "high" light output 64, a "middle" light output 65 and a "low" light output 66 in Figs. 4 (a) and (b) are the light output values which can be acquired after a predetermined number of years are passed from the shipment of the biological optical measurement instrument (for example, 3 years after the shipment).

Fig. 4 (a) shows the pattern in the case that the current which is injected upon acquiring the "high" light output 64, i.e. the current corresponding to the "high" light output 64 surpasses the absolute rated current and the warning for that condition is issued. The absolute rated current is stored in the storage unit 38 in advance.

When the current corresponding to the "high" light output 64 surpasses the absolute rated current, there is the risk that the light-emitting unit 16 is damaged and optical measurement can no longer be executed by the biological optical measurement instrument. The case that the current corresponding to the "high" light output 64 surpasses the absolute rated current is, for example the case, when the absolute rated current is set as 180(mA), that current I which is necessary for acquiring 2 (mW) that is the "high" light output 64 is: current I > 180 (mA) .

When the current corresponding to the "high" light output 64 surpasses the absolute rated current, the measurement/determination unit 44 determines that the light-emitting unit 16 needs to be replaced and displays the warning information on the display unit 36. An examiner or a service person of the instrument issues the command to replace the light-emitting unit 16 when he/she sees the warning information displayed on the display unit 36.

In the above description, since the current which surpasses the absolute rated current needs to be injected in order to confirm the fact that the current for acquiring the "high" light output 64 surpasses the absolute rated current, there is the risk that the light-emitting unit 16 may be damaged. Given this factor, the measurement/determination unit 44 may determine that the light-emitting unit 16 needs to be replaced and display the warning information on the display unit 36 even in the case that the light does not reach the "high" light output 64 at the time of applying the absolute rated current to the light-emitting unit 16, so as to avoid the current which surpasses the absolute rated current from being injected.

For example, in the case that the absolute rated current is set as 180(mA) and current I = 180(mA) is injected, the measurement/determination unit 44 measures whether the current reaches 2(mW) which is the "high" light output 64. When the current does not reach 2(mW) which is the "high" light output 64, the measurement/determination unit 44 determines that the light-emitting unit 16 needs to be replaced and causes the display unit 36 to display the warning information.

Fig. 4(b) shows the pattern that the attention is commanded in the case that the current corresponding to the "high" light output 64 is close to the absolute rated current, i.e. the case that the current corresponding to the "high" light output 64 is smaller than the absolute rated current for the portion of a predetermined value.

When the current corresponding to the "high" light output 64 reaches the current which is smaller than the absolute rated value for the portion of a predetermined value, the measurement/determination unit 44 determines that the light-emitting unit 16 needs to be replaced and causes the display unit 36 to display the information for attention. The case that the current corresponding to the "high" light unit 64 reaches a current value which is smaller than the absolute rated current for the portion of a predetermined value is, for example the case, when the absolute rated value is set as 180(mA), current I which is necessary for acquiring 2 (mW) that is the "high" light output 64 is: 160 (mA) < current I < 180(mA). At this time, the predetermined value is 20(mA).

When the current corresponding to the "high" light output 64 is sufficiently smaller than the absolute rated current, i.e. when the current corresponding to the "high" light output 64 does not reach the current which is smaller than the absolute rated value for the portion of a predetermined value, the measurement/ determination unit 44 determines that the light-emitting unit 16 can still be used, and displays this condition on the display unit 36. The case that the current corresponding to the "high" light output 64 is sufficiently smaller than the absolute rated current is, for example the case, when the absolute rated current is set as 180(mA), that current I which is necessary for acquiring 2 (mW) that is the "high" light output 64 is: current I < 160(mA).

Fig. 5 shows the pattern in which the warning is issued in the case that the light-output starting current is greater than a predetermined value. The light-output starting current is stored in the storage unit 38 in advance.

When the light-output starting current becomes greater than a predetermined value, there is the risk that the current corresponding to the "high" light output 64 surpasses the absolute rated current. The case that the light-output starting current becomes greater than a predetermined value is, for example when: light-output starting current I > 50 (mA). When the light-output starting current I is greater than a predetermined value, the measurement/determination unit 44 determines that the light-emitting unit 16 needs to be replaced, and causes the warning information to be displayed on the display unit 36.

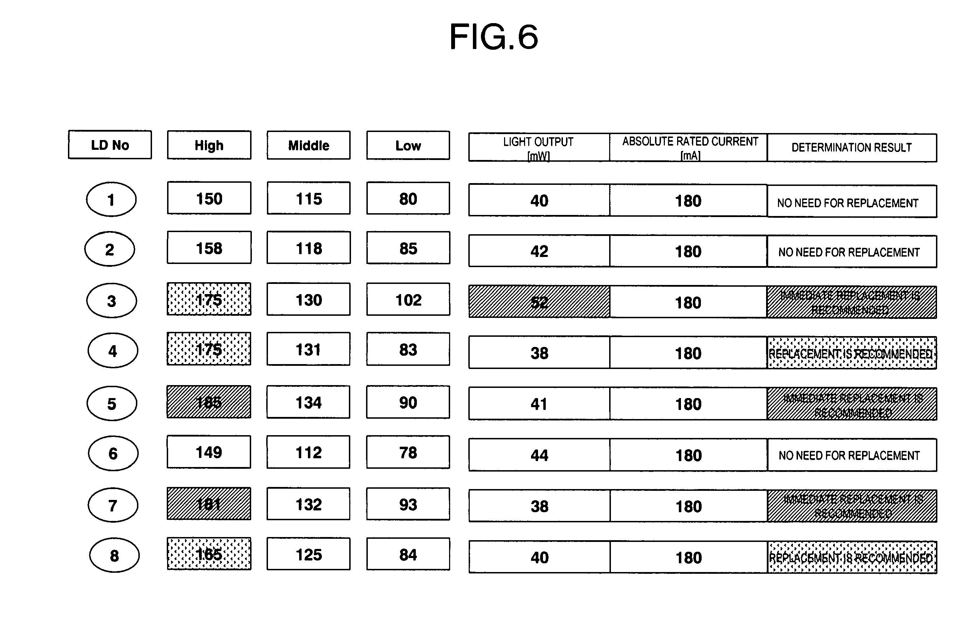

Fig. 6 is the display pattern of the display unit 36 wherein the current corresponding to the "high", "middle" and "low" light outputs, light-output starting current, injected current, absolute rated current and determination results are displayed corresponding to the number of the light-emitting unit (LD) 16.

For example, in the light-emitting unit 16 of No."1", the light-output starting current is smaller than the predetermined value (50(mA)) and the current corresponding to the "high" light output 64 is sufficiently smaller than the absolute rated current (180(mA)) (current I < 160(mA)), the information noting "no need for replacement" is displayed.

In the light-emitting unit of No."3", since the light-output starting current is greater than the predetermined value (50(mA)), the warning information noting "immediate replacement is necessary" is displayed. The display unit 36 prioritizes the display of warning information over the information for attention.

Also in the light-emitting unit 16 of No."8", since the current corresponding to the "high" light output 64 is smaller than the absolute rated current (180(mA)) and is greater than a current value which is smaller than the absolute rated value for the portion of the predetermined value (160(mA)), the information for attention noting "replacement is recommended" is displayed.

While the absolute rated current is set as 180(mA) in the above description, the operation unit 40 can arbitrarily change the absolute rated current or various predetermined values.

In the above description, when the current corresponding to the "high" light output 64 surpasses the absolute rated current, the measurement/determination unit 44 determined that the light-emitting unit 16 needs to be replaced and displayed the warning information on the display unit 36. In the present embodiment, in the case that the current corresponding to the "middle" light output 65 and the "low" light output 66 of which the light output is smaller than the "high" light output 64 is smaller than the absolute rated value, the measurement/determination unit 44 may determine that the light-emitting unit 16 can be used by the "middle" light output 65 and the "low" light output 66 and display the information noting on the display unit 36 that there is no need for replacement if the light output is the "middle" light output 65 or the "low" light output 66. The case that the current corresponding to the "middle" light output 65 is smaller than the absolute rated current is the case, for example when the absolute rated current is set as 180(mA), that current I which is necessary for acquiring 1(mW) which is the "middle" light output 65 is: current I < 180(mA).

Also, while the information to command warning or attention is displayed on the display unit 36 in the above description, the information may also be sent to the examiner or service person by sound, etc. Further, the information for warning or attention may be set to be sent automatically to a personal computer or mobile telephone owned by the manufacturer of the light-emitting unit 16 via the control unit 14 or the network of the biological optical measurement instrument.

As mentioned above, in accordance with the present embodiment, an operator or a service person can detect the deterioration state and necessity/unnecessity of replacing the light-emitting unit 16, and command replacement of the light-emitting unit 16 at proper timings.

Embodiment 2

(Graph Comparison)

Here, the second embodiment will be described referring to Fig. 1 ~ Fig. 7. The difference of the present embodiment from the first embodiment is that the measurement/determination unit 44 determines the state of the light-emitting unit 16 based on the characteristic of the graph created by the light output (mW) of the light-emitting unit 16 and the current (mA) injected into the light-emitting unit 16.

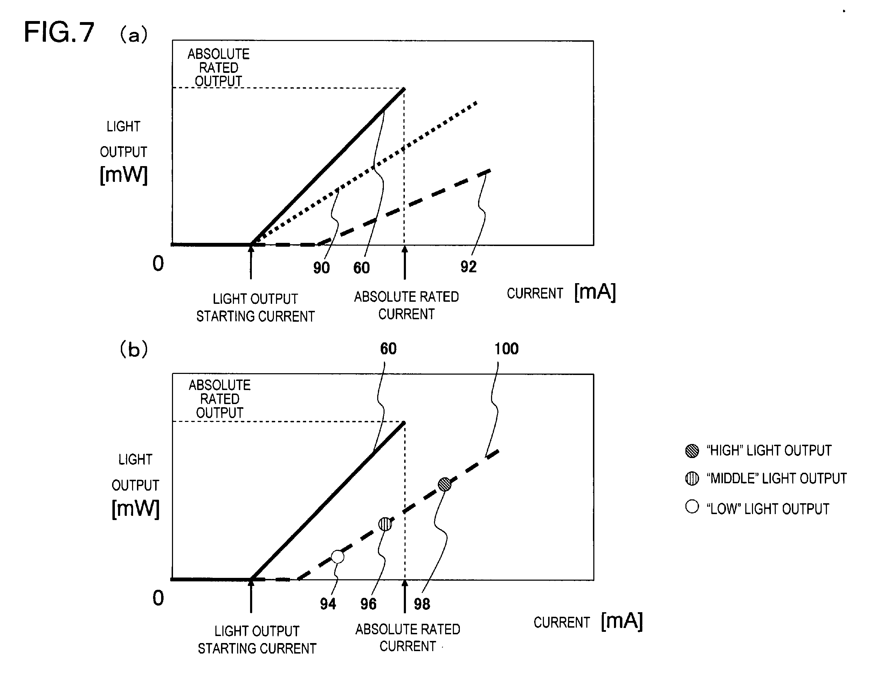

In Fig. 7(a), the measurement/determination unit 44 measures the deterioration state of the light-emitting unit 16 from the slope of graphs 90 and 92 which are created on the basis of the light output of the light-emitting unit 16 and the current injected into the light-emitting unit 16, and determines the necessity/unnecessity of replacing the light-emitting unit 16 based on the deterioration state. The display unit 36 displays the deterioration state and the necessity/unnecessity of replacement of the light-emitting unit 16.

The graph 60 shows the relationship between the light output and the current at the shipment time of the biological optical measurement instrument, i.e. in a normal condition. The graph 90 is based on the light output and the current acquired after a certain number of years have passed since the shipment (for example, 2 years after the time of shipment) of the biological optical measurement instrument, and the graph 92 is based on the light output and the current acquired after some years have passed since the time of the shipment (for example, 6 years after the shipment).

The light-emitting unit 16 has the tendency that the slope of the graph based on the light output and the current of the light-emitting unit 16 gradually becomes smaller with the passing of time since the time of shipment. The measurement/determination unit 44 makes determination, when the slope of the graph based on the light output and the current of the light-emitting unit becomes smaller than a predetermined value, to replace the light-emitting unit 16, and displays the warning information on the display unit 36 by using this characteristic.

In Fig. 7(b), the measurement/determination unit 44 obtains a graph 100 from at least two pairs of the light output and the current corresponding to the relevant light output, and determines the necessity/unnecessity of replacement of the light-emitting unit 16 from the obtained graph 100. The display unit 36 displays the deterioration state and the necessity/unnecessity of replacement of the light-emitting unit 16.

The measurement/determination unit 44 creates the graph 100 which is the straight line passing through two points based on a "low" light output 94, a "middle" light output 96 and the current corresponding to the relevant light output. The measurement/determination unit 44 determines that the light-emitting unit 16 needs to be replaced when the slope of the graph 100 based on the light output and the current reaches less than a predetermined value, and causes the warning information to be displayed on the display unit 36.

Also, the measurement/determination unit 44 marks a "high" light output 98 in the graph 100. When the current corresponding to the "high" light output 98 in the a straight line 100 surpasses the absolute rated current, the measurement/determination unit 44 determines that the light-emitting unit 16 needs to be replaced, and causes the warning information to be displayed on the display unit 36. An examiner or a service person issues an instruction to replace the light-emitting unit 16 when he/she sees the warning information displayed on the display unit 36.

While the measurement/determination unit 44 creates the graph 100 of the straight line passing through two points in the above description, the graph 100 of a straight line may be created using more than three points. Also, the measurement/determination unit 44 may also create the graph 100 of a straight line or a curved line using the method of least squares, etc.

As described above, in accordance with the present embodiment, an examiner or a service person can detect the deterioration state or the necessity/unnecessity of replacement of the light-emitting unit 16, and issues an instruction to replace the light-emitting unit 16 at the proper time.

Embodiment 3

(Estimation of Life Duration)

Here, the third embodiment will be described using Fig.1 - Fig. 8. The difference of the present embodiment from the first and the second embodiments is that the measurement/determination unit 44 estimates usable life of the light-emitting unit 16.

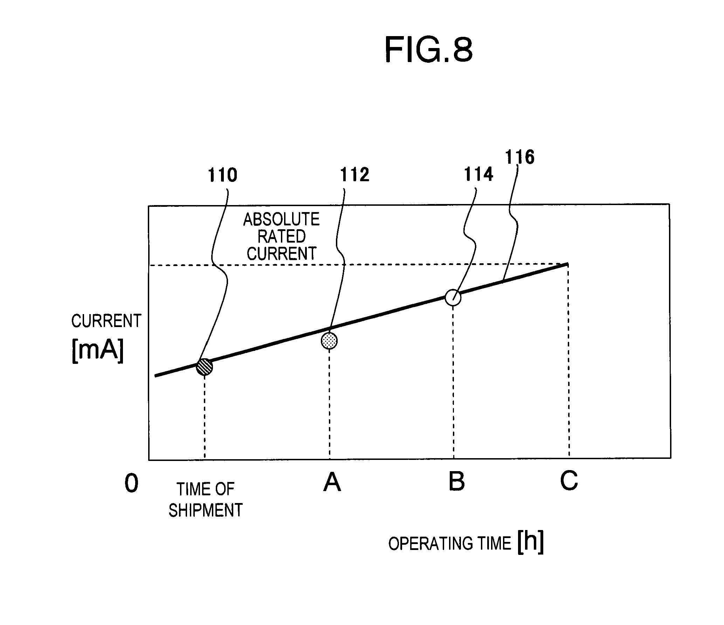

Fig. 8 shows the pattern of measuring the current injected upon acquiring a "high" light output at the time of shipment, time A and time B. Time A is when a certain number of years have passed since the time of shipment (for example, 2 years from the time of shipment) and time B is when some years are further passed since time A (for example, 4 years since the time of shipment).

When the current point is set as time B, the measurement/determination unit 44 reads out from the storage unit 38 a current 110 injected upon acquiring a "high" light output at the time of shipment with time information, and obtains from the power meter 42 a current 114 injected upon acquiring the "high" light output 64 at the current time B. Then a graph 116 showing the relationship between the currents 110 and 114 and the time is created. At this time, the measurement/determination unit 44 may also create a graph 116 using a current 112 at time A and the time information.

Then the measurement/determination unit 44 calculates the timing that the graph 116 reaches the absolute rated current as time C. Also, the measurement/determination unit 44 calculates the estimated usable period (time C - time B) at time B. The display unit 36 displays the estimated usable life and the time that the current reaches the absolute rated current (for example, the date and time). The examiner or service person can see the estimated usable period and the timing that the current reaches the absolute rated current which are displayed on the display unit 36, and determine how long the light-emitting unit 16 can be used after time B and when to issue an instruction for the replacement thereof.

While the current to be injected upon acquiring the "high" light output is calculated which is the light output to be used for a normal measurement mode in the above description, the current to be injected upon acquiring the "middle" and "low" light outputs which are the measurement modes for reduced light outputs may also be calculated.

As described above, in accordance with the present embodiment, an examiner or service person can detect the deterioration state of the light-emitting unit 16, the timing that the current reaches the absolute rated current and the estimated usable period of the light-emitting unit 16 and determine the proper timing to replace the light-emitting unit 16.

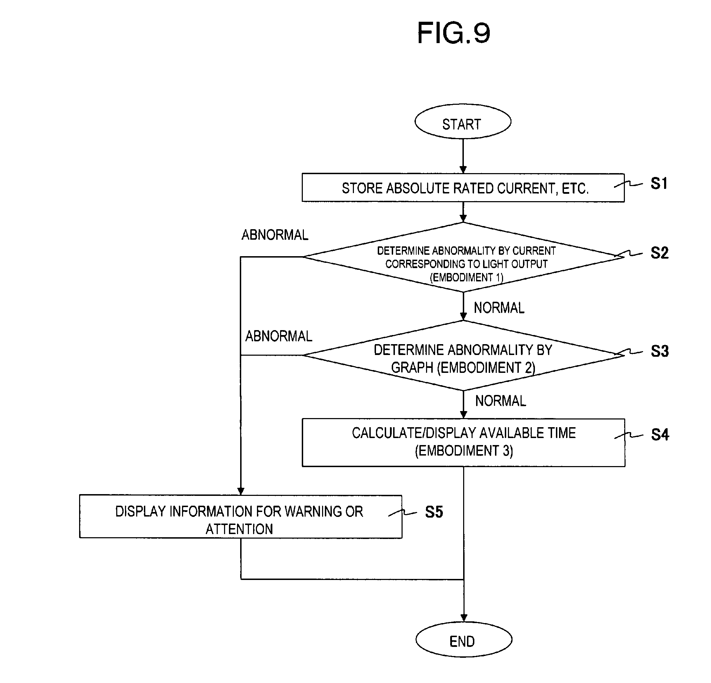

Here, the operation in the first ~ third embodiments of the present invention will be described referring to Fig. 9.

(step 1)

At the time of shipment of an optical measurement instrument, the light output which is outputted from the light power meter 42 and the current to be injected into the light-emitting unit 16 which is necessary for the light output are stored in the storage unit 38 along with the time information on the shipment (for example, the date and time). This means that the information of graph 60 in Fig. 4 which has the characteristics that the more the current is increased from the light-output starting current the grater the light output becomes is also stored in the storage unit 38.

Also, when the examiner or service person determines the absolute rated current or light-output starting current in the first embodiment and the slope of a graph or parameters such as various predetermined values in the second embodiment, he/she inputs the parameters into the operating unit 40. The inputted parameters are stored in the storage unit 38 via the control unit 14.

(step 2)

The measurement/determination unit 44 determines whether the current corresponding to the "high" light output 64 which is the normal light output to be used in the normal measurement mode surpasses the absolute rated current. In other words, the first embodiment is carried out. When the relevant current surpasses the absolute rated current, the determination is made as abnormal (deteriorated), and step 5 is carried out. When the relevant current does not surpass the absolute rated current, the determination is made as normal and step 3 is carried out.

(Step 3)

The measurement/determination unit 44 creates the graphs 90, 92 and 100 on the basis of the currently acquired light output and the current. The measurement/determination unit 44 determines whether the slope of the graphs 90, 92 and 100 has a smaller value than a predetermined value. In other words, the second embodiment is carried out. When the relevant slope has a value which is smaller than the predetermined value, the determination is made as abnormal and step 5 is carried out. When the relevant slope has a value smaller than the predetermined value, the determination is made as normal, and step 4 is carried out.

(Step 4)

The measurement/determination unit 44 reads out from the storage unit 38 a current 110 injected upon acquiring the "high" light output at the time of shipment along with the time information, and obtains a current 114 injected upon acquiring the current "high" light output 64 from the power meter 42. Then a graph 116 showing the relationship between the currents 110 and 114 and the time is created. The measurement/determination unit 44 calculates the timing that the graph 116 reaches the absolute rated current and the estimated usable period, and the display unit 36 displays the timing that the current reaches the calculated absolute rated current or the estimated usable period. In other words, the third embodiment is carried out.

(Step 5)

When the determination is made as abnormal in step 2 or step 3, the measurement/determination unit 44 causes the warning information or attention information for recommending replacement of the light-emitting unit 16 to be displayed on the display unit 36. When the examiner or service person sees the warning information or attention information displayed on the display unit 36, he/she issues an instruction to replace the light-emitting unit 16.

Embodiment 4

(Gain Value Comparison)

Here, the fourth embodiment will be described referring to Fig. 1 ~ Fig. 11. The difference of the present embodiment from the first ~ third embodiments is that the measurement/determination unit 44 measures the deterioration state of the light-emitting unit 16 from the gain value set by the gain adjustment unit 33 and determines the necessity/ unnecessity of the light-emitting unit 16.

In the present embodiment, the irradiating optical fibers 20 and the detecting optical fibers 26 are applied not to the object 22 but to an inspection holder (not shown in the diagram) having a light-detecting inspection body of a biological simulation sample (phantom). This is for suppressing the fluctuation of the gain value that varies depending on a measuring position in the object 22. In the present embodiment, the optical fibers without disconnection are used for the irradiating optical fibers 20 and the detecting optical fibers 26.

The gain adjustment unit 33 multiplies the digital signal outputted from the A/D converter 32 by the gain value on the basis of the electric signal based on the light quantity of the transmitted light detected by a plurality of photoelectric transducers 28.

The gain value is defined to uniformize digital signals. The gain value improves the S/N ratio and secures the reliability of digital signals. In concrete terms, the gain adjustment unit 33 multiplies the digital signal by the gain value, so that the detected light quantity of the plurality of photoelectric transducers 28 is uniformized, for example at 2(m"). When the detected light quantity is small (for example, 2(mW) or smaller) the gain value by which the digital signal is multiplied becomes greater (for example, 1 or greater), and when the detected light quantity is great (for example, 2 (mW) or greater) the gain value by which the digital signal is multiplied becomes smaller (for example, 1 or less). The gain value obtained by the gain adjustment unit 33 is stored in the storage unit 38 with the time information. The above description is the case of the "high" light output.

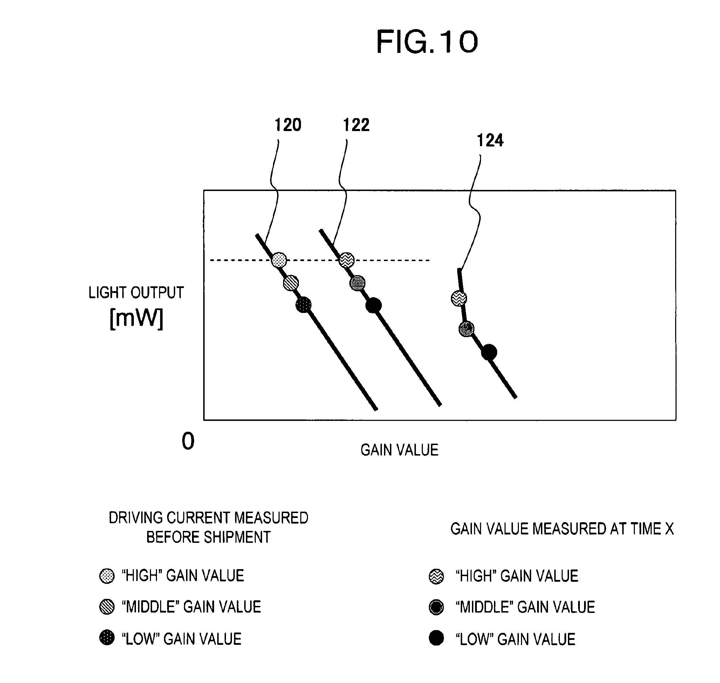

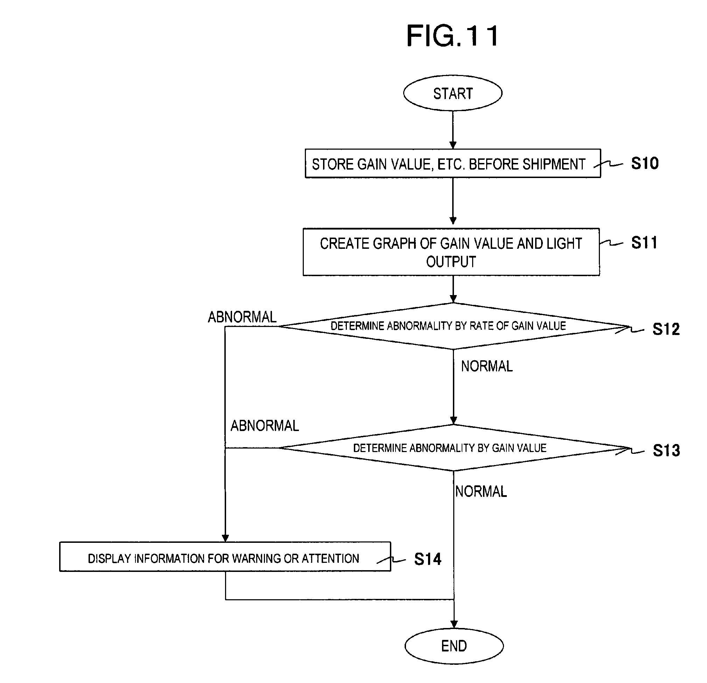

Fig. 10 shows the pattern that the gain value which is to be multiplied in the gain adjustment unit 33 is measured at the time of shipment and time X(present). Time X is when a certain years passed since the time of shipment (for example, 3 years after the shipment). Fig. 11 shows the operation of the present embodiment which describes the operation of the present embodiment.

(Step 10)

At the shipment time of the biological optical measurement instrument, the light output which is outputted from the light power meter 42 and the gain value are stored in the storage unit 38 along with the time information (for example, the date and time) at the time of the shipment. Also, when the examiner or service person determines the parameters of the threshold value or the ratio of the gain value to be described later, etc., the examiner or service person inputs the parameters in the operation unit 40. The parameters are stored in the storage unit 38 via the control unit 14.

(Step 11)

The measurement/determination unit 44 reads out the gain value stored at the time of shipment and the light output of the light-emitting unit 16 from the storage unit 38, and creates a graph 120. The display unit displays the graph 120.

(Step 12)

The respective gain values obtained by adjusting the light output of the light-emitting unit 16 to ""high"", "Normal" and "Low" at the time of shipment are set as "Ho", "No" and "Lo". Also, when the ratio of the respective gain values are set as "$A_H$", "$A_N$" and "$A_L$" at the time that the respective gain values obtained at time X are set as "Hx", "Nx" and "Lx", the following equation can be obtained.

[Equation 1]

$$A_H = \frac{H_x}{H_o} \quad , \quad A_N = \frac{N_x}{N_o}, \quad A_L = \frac{L_x}{L_o}$$

The measurement/determination unit 44 determines whether the 3 values of the ratio $A_H$, AN and $A_L$ are equal. If these values are not equal, the same characteristics as in the graph 120 shown in Fig. 10 cannot be retained. For example, when the graph such as a graph 124 is obtained, the measurement/determination unit 44 determines that the light-emitting unit 16 is abnormal (deteriorated). When the relevant ratio $A_H$, AN and $A_L$ are not equal, the determination is made as abnormal and step 14 is carried out. When the relevant ratio $A_H$, AN and $A_L$ are equal, the determination is made as normal and step 13 is carried out.

(Step 13)

The measurement/determination unit 44 determines the deterioration state of the light-emitting unit 16 on the basis of the respective gain values Hx, Nx and Lx obtained at time X. In concrete terms, when any of the respective gain values Hx, Nx and Lx becomes more than the threshold value (for example, 2 or more), the measurement/determination unit 44 determines that the light-emitting unit 16 is abnormal. When any of the relevant respective gain values Hx, Nx and Lx becomes more than the threshold value, the determination is made as abnormal and step 14 is carried out. When any of the relevant respective gain values Hx, Nx and Lx becomes less than the threshold value, the determination is made as normal and the biological optical measurement is continued.

(Step 14)

When there is at least one light-emitting unit 16 which is determined as abnormal, a warning/attention screen is displayed on the display unit 36. On the warning/attention screen, a comment such as "a service person needs to contact the manufacturer of the light-emitting unit 16" or the name of the service person or the person in charge of the manufacturer is displayed. When the examiner or service person sees a warning/attention screen 80, he/she issues the instruction to replace the light-emitting unit 16.

In accordance with the present embodiment, it is possible to determine the necessity/unnecessity of replacing the light-emitting unit 16 on the basis of only the gain value obtained from the biological optical measurement instrument without using the power meter 42, and so on. Therefore, the examiner can detect the deterioration state and the necessity/unnecessity of the light-emitting unit 16 without depending on the service person.

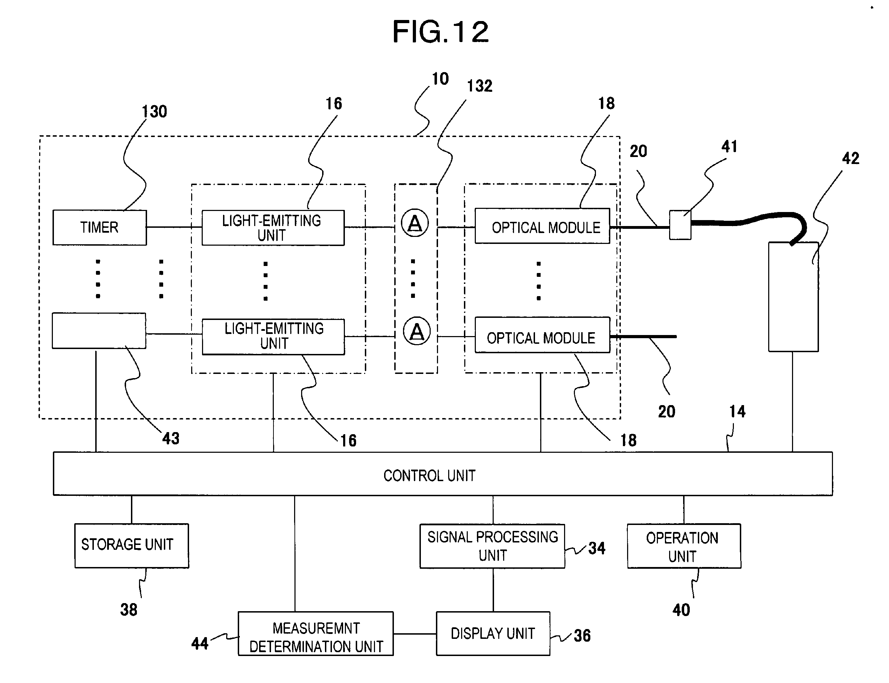

Fig. 12 shows the optional pattern in which a timer 130 and an ammeter 132 are attached to the light-emitting unit 16. The following pattern can be applied to the above-described first - fourth embodiments.

If the timer 130 is operated when the light-emitting unit 16 emits light, the driving time of the light-emitting unit 16 can be accurately detected by the timer 130. The years/months/days and time from the time of shipment also can be measured. Also, the driving time which can be acquired from the timer 130 and the record of the light output of the respective light-emitting units 16 can be stored in the storage unit 38.

Also, the function to measure the current, for example the ammeter 132 can be provided between the light-emitting unit 16 and the optical module 18 in the light source unit 10. The injected current of the light-emitting unit 16 can be directly measured by the ammeter 132 without using a device such as the power meter 42. Therefore, the current can be measured without considering the damage of irradiating optical fiber 20.

In the case that the sufficient light output cannot be acquired at the end of irradiating optical fiber 20 even though the current to be injected into the light-emitting unit 16 is increased, it is necessary to confirm the disconnection state of the irradiating optical fiber 20.

In accordance with the above-described embodiment, an examiner or service person can detect the deterioration state or the necessity/unnecessity of replacing a light-emitting unit and issue an instruction to replace the light-emitting unit at a proper timing.

Description of Reference Numerals

10: light source unit
12: light measurement unit
14: control unit
16: light-emitting unit (light-emitting diode)
18: optical module
20: irradiating optical fiber
22: object
23: probe holder
26: detecting optical fiber
28: photoelectric transducer
30: lock-in amplifier module
32: A/D converter
33: gain adjustment unit
34: signal processing unit
36: display unit
38: storage unit
40: input/output unit
44: measurement/determination unit

**Claims**

1. A biological optical measurement instrument comprising:

   a light source unit which has light-emitting units that emit light, configured to irradiate the light to an object to be examined;
   a light measurement unit configured to measure the transmitted light at a measurement point in the object;
   a signal processing unit configured to process the hemoglobin information outputted from the light measurement unit and create a light measurement image; and
   a display unit configured to display the light measurement image,
   further comprising a measurement/determination unit configured to measure the deterioration state of the light-emitting unit based on the light output of the light-emitting unit and the current injected into the light-emitting unit, and determines the necessity/unnecessity of replacement of the light-emitting unit,
   wherein the display unit displays the information on the necessity/unnecessity of replacing the light-emitting unit which is determined by the measurement/determination unit.

2. The biological optical measurement instrument according to claim 1, wherein the measurement/determination unit, when the current injected into the light-emitting unit surpasses the absolute rated current, determines that the light-emitting unit needs to be replaced.

3. The biological optical measurement instrument according to claim 1, wherein the measurement/determination unit, when the light output does not reach a predetermined value at the time that the absolute rated current is injected into the light-emitting unit, determines that the light-emitting unit needs to be replaced.

4. The biological optical measurement instrument according to claim 1, wherein the measurement/determination unit, when the current injected into the light-emitting unit reaches a current value which is smaller than the absolute rated value for the portion of a predetermined value, determines that the light-emitting unit needs to be replaced.

5. The biological optical measurement instrument according to claim 1, wherein the measurement/determination unit, when the current injected into the light-emitting unit does not reach a current value which is smaller than the absolute rated current for the portion of a predetermined value, determines that the light-emitting unit does not need to be replaced.

6. The biological optical measurement instrument according to claim 1, wherein the measurement/determination unit, when the light-output starting current at which the light output of the light-emitting unit is started is greater than a predetermined value, determines that the light-emitting unit needs to be replaced.

7. The biological optical measurement instrument according to claim 1, wherein the measurement/determination unit, when the current corresponding to a light output which is smaller than the light output is smaller than the absolute rated current, determines that the light-emitting unit does not need to be replaced if the light output is the previously mentioned small light output.

8. The biological optical measurement instrument according to claim 1, wherein the measurement/determination unit, when the value of slope in a graph based on the light output and the current of the light-emitting unit reaches smaller than a predetermined value, determines that the light-emitting unit needs to be replaced.

9. The biological optical measurement instrument according to claim 8, wherein the measurement/determination unit marks a predetermined light output in the graph, and determines, when the current corresponding to a predetermined light output surpasses the absolute rated current, that the light-emitting unit needs to be replaced.

10. The biological optical measurement instrument according to claim 1, comprising a usable-period calculation unit configured to calculate the timing that the current reaches the absolute rated current and the usable period according to the graph based on the current corresponding to the light output of the light-emitting unit and time, wherein the measurement/determination unit determines the necessity/unnecessity of replacement of the light-emitting unit on the basis of the timing that the current reaches the absolute rated current and the estimated usable period.

11. The biological optical measurement instrument according to claim 1, comprising a light-emitting unit condition measuring unit configured to measure the deterioration state of the light-emitting unit on the basis of the gain value which is set, on the basis of the light quantity of the transmitted light measured by the light emitting unit, by the gain adjustment unit that sets a gain value by which the digital signal based on the hemoglobin information is multiplied, wherein the measurement/determination unit determines the necessity/unnecessity of replacement of the light-emitting unit from the deterioration state thereof.

12. The biological optical measurement instrument according to claim 11, wherein light-emitting unit condition measuring unit measures the deterioration state of the light-emitting unit by the ratio between the gain value at the time of shipment and the current gain value.

13. The biological optical measurement instrument according to claim 1, comprising an ammeter configured to measure a current, between an optical module provided with a modulator that modulates the light emitted by the light-emitting unit by a plurality of different frequencies and the light-emitting unit.

14. The biological optical measurement instrument according to claim 1, wherein the light-emitting unit emits near-infrared light that includes a semiconductor laser or light-emitting diode.

15. A method for displaying information regarding the necessity/unnecessity of replacement of a light-emitting unit including steps of:

measuring the deterioration state of a light-emitting unit from the light output of the light-emitting unit that emits light and the current injected into the light-emitting unit;
determining the necessity/unnecessity of replacing the light-emitting unit from the deterioration state; and
displaying the information regarding necessity/unnecessity of replacement of the light-emitting unit.

# FIG.1

EP 2 502 567 A1

# FIG.2

OPERATION UNIT 40

42

41

20

20

10

18

OPTICAL MODULE 18

OPTICAL MODULE 18

16

LIGHT-EMITTING UNIT 16

LIGHT-EMITTING UNIT 16

14

CONTROL UNIT

SIGNAL PROCESSING UNIT 34

DISPLAY UNIT 36

MEASUREMNT/ DETERMINATION UNIT 44

STORAGE UNIT 38

# FIG.3

EP 2 502 567 A1

# FIG.4 (a)

ABSOLUTE RATED OUTPUT

LIGHT OUTPUT [mW]

63  62  61  60

⬤ "HIGH" LIGHT OUTPUT

◍ "MIDDLE" LIGHT OUTPUT

○ "LOW" LIGHT OUTPUT

0

66  65  64

CURRENT [mA]

LIGHT OUTPUT STARTING CURRENT

ABSOLUTE RATED CURRENT

## (b)

70

ABSOLUTE RATED OUTPUT

LIGHT OUTPUT [mW]

⬤ "HIGH" LIGHT OUTPUT

◍ "MIDDLE" LIGHT OUTPUT

○ "LOW" LIGHT OUTPUT

0

66  65  64

CURRENT [mA]

LIGHT OUTPUT STARTING CURRENT

ABSOLUTE RATED CURRENT

EP 2 502 567 A1

# FIG.5

ABSOLUTE RATING

DRIVING OUTPUT

LIGHT OUTPUT [mW]

60    75

0    CURRENT [mA]

LIGHT OUTPUT
STARTING CURRENT

LIGHT OUTPUT
STARTING CURRENT

EP 2 502 567 A1

# FIG.6

| LD No | High | Middle | Low | LIGHT OUTPUT [mW] | ABSOLUTE RATED CURRENT [mA] | DETERMINATION RESULT |
|:---:|:---:|:---:|:---:|:---:|:---:|:---:|
| 1 | 150 | 115 | 80 | 40 | 180 | NO NEED FOR REPLACEMENT |
| 2 | 158 | 118 | 85 | 42 | 180 | NO NEED FOR REPLACEMENT |
| 3 | 175 | 130 | 102 | 52 | 180 | IMMEDIATE REPLACEMENT IS RECOMMENDED |
| 4 | 175 | 131 | 83 | 38 | 180 | REPLACEMENT IS RECOMMENDED |
| 5 | 185 | 134 | 90 | 41 | 180 | IMMEDIATE REPLACEMENT IS RECOMMENDED |
| 6 | 149 | 112 | 78 | 44 | 180 | NO NEED FOR REPLACEMENT |
| 7 | 181 | 132 | 93 | 38 | 180 | IMMEDIATE REPLACEMENT IS RECOMMENDED |
| 8 | 165 | 125 | 84 | 40 | 180 | REPLACEMENT IS RECOMMENDED |

EP 2 502 567 A1

FIG.7 (a)

FIG.7 (b)

⬤ "HIGH" LIGHT OUTPUT

◍ "MIDDLE" LIGHT OUTPUT

◯ "LOW" LIGHT OUTPUT

EP 2 502 567 A1

# FIG.8

EP 2 502 567 A1

# FIG.9

```
        ┌─────────┐
        │  START  │
        └────┬────┘
             │
             ▼
   ┌──────────────────────────────────┐
   │  STORE ABSOLUTE RATED CURRENT, ETC.│─── S1
   └────────────────┬─────────────────┘
                    │
                    ▼
ABNORMAL   ◇─────────────────────────◇
◄──────────  DETERMINE ABNORMALITY BY CURRENT ─── S2
           CORRESPONDING TO LIGHT OUTPUT
                (EMBODIMENT 1)
                    │ NORMAL
                    ▼
ABNORMAL   ◇─────────────────────────◇
◄──────────  DETERMINE ABNORMALITY BY   ─── S3
             GRAPH (EMBODIMENT 2)
                    │ NORMAL
                    ▼
        ┌──────────────────────────────┐
        │ CALCULATE/DISPLAY AVAILABLE TIME│─── S4
        │       (EMBODIMENT 3)           │
        └────────────────┬─────────────┘
                         │
  ┌──────────────────────────────┐
  │ DISPLAY INFORMATION FOR WARNING OR │─── S5
  │          ATTENTION             │
  └──────────────────────────────┘
                         │
                         ▼
                    ┌─────────┐
                    │   END   │
                    └─────────┘
```

EP 2 502 567 A1

# FIG.10

LIGHT OUTPUT
[mW]

120  122  124

0  GAIN VALUE

DRIVING CURRENT MEASURED
BEFORE SHIPMENT

◉ "HIGH" GAIN VALUE

◉ "MIDDLE" GAIN VALUE

● "LOW" GAIN VALUE

GAIN VALUE MEASURED AT TIME X

◉ "HIGH" GAIN VALUE

◉ "MIDDLE" GAIN VALUE

● "LOW" GAIN VALUE

# FIG.11

START

STORE GAIN VALUE, ETC. BEFORE SHIPMENT — S10

CREATE GRAPH OF GAIN VALUE AND LIGHT OUTPUT — S11

DETERMINE ABNORMALITY BY RATE OF GAIN VALUE — S12

ABNORMAL

NORMAL

DETERMINE ABNORMALITY BY GAIN VALUE — S13

ABNORMAL

NORMAL

DISPLAY INFORMATION FOR WARNING OR ATTENTION — S14

END

# FIG.12

EP 2 502 567 A1

<table>
<tr><td colspan="2" align="center">**INTERNATIONAL SEARCH REPORT**</td><td>International application No.<br>PCT/JP2010/070079</td></tr>
</table>

A. CLASSIFICATION OF SUBJECT MATTER
*A61B10/00*(2006.01)i, *A61B5/1455*(2006.01)i

According to International Patent Classification (IPC) or to both national classification and IPC

B. FIELDS SEARCHED

Minimum documentation searched (classification system followed by classification symbols)
A61B10/00, A61B5/1455

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2009-22353 A (Hitachi, Ltd.),<br>05 February 2009 (05.02.2009),<br>fig. 11<br>(Family: none) | 1-5,7,10,<br>13-15 |
| Y | JP 2002-310669 A (Hitachi Cable, Ltd.),<br>23 October 2002 (23.10.2002),<br>(Family: none) | 1-5,7,10,<br>13-15 |
| Y | JP 9-8277 A (The Tokyo Electric Power Co.,<br>Inc.),<br>10 January 1997 (10.01.1997),<br>(Family: none) | 1-5,7,10,<br>13-15 |
| Y | JP 2000-31542 A (The Nippon Signal Co., Ltd.),<br>28 January 2000 (28.01.2000),<br>(Family: none) | 1-5,7,10,<br>13-15 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>24 December, 2010 (24.12.10) | Date of mailing of the international search report<br>11 January, 2011 (11.01.11) |
|---|---|
| Name and mailing address of the ISA/<br>Japanese Patent Office | Authorized officer |
| Facsimile No. | Telephone No. |

Form PCT/ISA/210 (second sheet) (July 2009)

25

**INTERNATIONAL SEARCH REPORT**

International application No.

PCT/JP2010/070079

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2004-296841 A  (Seiko Epson Corp.),<br>21 October 2004 (21.10.2004),<br>(Family: none) | 1-5,7,10,<br>13-15 |
| A | JP 2001-137247 A  (Hitachi Medical Corp.),<br>22 May 2001 (22.05.2001),<br>paragraph [0025]; fig. 1<br>(Family: none) | 1-15 |
| A | JP 2007-209680 A  (Hitachi Medical Corp.),<br>23 August 2007 (23.08.2007),<br>paragraphs [0014] to [0017]<br>(Family: none) | 1-15 |
| A | JP 2008-227463 A  (Seiko Epson Corp.),<br>25 September 2008 (25.09.2008),<br>paragraph [0042]<br>& US 2008/191701 A1 | 1-15 |
| A | JP 62-274234 A  (Hitachi, Ltd.),<br>28 November 1987 (28.11.1987),<br>(Family: none) | 1-15 |

Form PCT/ISA/210 (continuation of second sheet) (July 2009)

**EP 2 502 567 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007209680 A